# EUROPEAN PATENT SPECIFICATION

(11) **EP 0 938 437 B1**
(45) Date of publication and mention of the grant of the patent: **17.01.2001**
(21) Application number: 97908622.0
(22) Date of filing: 04.03.1997
(51) Int. Cl.: B65D 85/16, A61F 13/15, B65B 63/02

(54) **FLEXIBLE PACKAGING UNIT CONTAINING FOLDED ABSORBENT ARTICLES**
FLEXIBLE PACKUNG MIT GEFALTETEN SAUGFÄHIGEN ARTIKELN
CONDITIONNEMENT FLEXILE CONTENANT DES ARTICLES ABSORBANTS PLIES

(30) Priority: 15.03.1996 SE 9600996
(43) Date of publication of application: 01.09.1999
(60) Divisional application: 99109918.5
(73) Proprietor: SCA Hygiene Products AB, 405 03 Göteborg (SE)
(72) Inventor: MATTIASSON, Marina, S-416 67 Göteborg (SE)
(74) Representative: Hyltner, Jan-Olof
(86) International application number: SE9700371
(87) International publication number: WO9733815

(56) References cited:
- EP-A- 0 225 865
- EP-A- 0 419 770
- WO-A-96/20884
- GB-A- 2 273 279
- US-A- 3 325 003
- US-A- 4 770 298
- US-A- 5 036 978
- US-A- 5 163 558

## Description

The present invention relates to a flexible packaging unit containing at least one compressed bundle of absorbent articles, such as diapers or incontinence guards, each comprising a rear part, a front part, an intermediate crotch part, and an absorbent body enclosed between enveloping casing sheets, as defined in the preamble of claim 1.

Absorbent articles of the aforesaid kind are normally folded together to form a rectangular shape and then double-folded prior to being packaged. One drawback with folding the articles in this way, is that folding is effected in the crotch part of the article, which is often the most sensitive part of the absorbent body. After package-folding the articles, the articles are bundled and then compressed so as to reduce the bundle volume, prior to inserting the bundle into a bag or like packaging unit made of flexible material. This compression significantly reduces the volume of the bundle of articles. The packaged articles therewith take up much less transport space and storage space than would be the case if the articles were not compressed. However, the compressed packaged articles strive to return to their original thickness and therewith press against the walls of the package and against each other with a relatively large force. The pressure thus exerted by the packaged articles can make it difficult to remove the first articles from a package. This problem has been solved traditionally, by providing one of the walls of the package with relatively large tear-away pieces; see for instance EP-A2-0,349,050 and EP-BI-0,554,411. Removal of these relatively large tear-away pieces leaves relatively large openings through which parts of package-folded articles are able to fan-out, because expansion of these parts of the articles can no longer be prevented by the package wall. An opened package will therewith require more space than an unopened package.

The object of the present invention is to eliminate the aforesaid drawbacks and to provide a packaging unit ,for compressed absorbent articles in which the articles are positively retained even when the package is opened, and from which articles can be easily taken.

These objects are achieved in accordance with the invention by means of a flexible packaging unit for accommodating at least one compressed bundle of absorbent articles, such as diapers or incontinence guards, each including a rear part, a front part, an intermediate crotch part and an absorbent body enclosed between enveloping casing sheets, said articles being folded so that outer portions of the front and the rear parts will be folded-in towards the crotch part, at least in the longitudinal direction, and face in one and the same direction in a respective bundle, said packaging unit embracing the bundle or stack of articles and having a generally parallelepipedic shape that includes an upper side, a lower side, two mutually opposing side walls and two mutually opposing end walls, and package-unit opening means which enable the unit to be opened so that articles can be taken therefrom, wherein the packaging unit is characterized in that the package-unit opening means comprises an opening-forming part on the end wall towards which the inwardly folded portions of the front and rear parts of the articles in said bundle face, wherein the opening-forming part extends parallel with the front and rear edges of the inwardly folded front and rear article parts and can be moved out of the plane of said end wall and has a position and a width such that at least one of the outer edges of the front or the rear article part will be exposed when the opening-forming part is moved out of the plane of said end wall.

In a preferred embodiment, at least one of the front and the rear article parts includes a longitudinally outer edge-part of casing material located outwardly of the absorbent body, said edge-part overlapping a portion of the upper of said front or rear article parts in the packaged-folded state of the article, wherein said opening-forming part extends along the edge of said outer edge-part of the casing material. The opening-forming part has a smallest width of 10 mm and a largest width corresponding to 33% of the length of a package-folded article and is defined by two mutually parallel weakening lines that extend over the full width of the end wall, from one side wall to the other. The parallel weakening lines extend slightly into the side walls and a further weakening line is provided in one of the side walls, between the ends of the parallel weakening lines.

The invention will now be described in more detail with reference to the accompanying drawings, in which
Figs. 1 to 4 are schematic views illustrating the different steps in package-folding a diaper in accordance with the invention from above;
Fig. 5 is a side view of the diaper shown in Fig. 4;
Figs. 6 and 7 illustrate schematically the introduction of a bundle of package-folded diapers into a packaging unit according to a first embodiment of the invention;
Fig. 8 is a schematic perspective view of an opened packaging unit;
Figs. 9 to 12 are schematic side views with one side wall removed and show the different stages of removing a diaper from an opened packaging unit; and
Fig. 13 illustrates an end wall provided with an opening-forming means in accordance with a second embodiment of the invention.

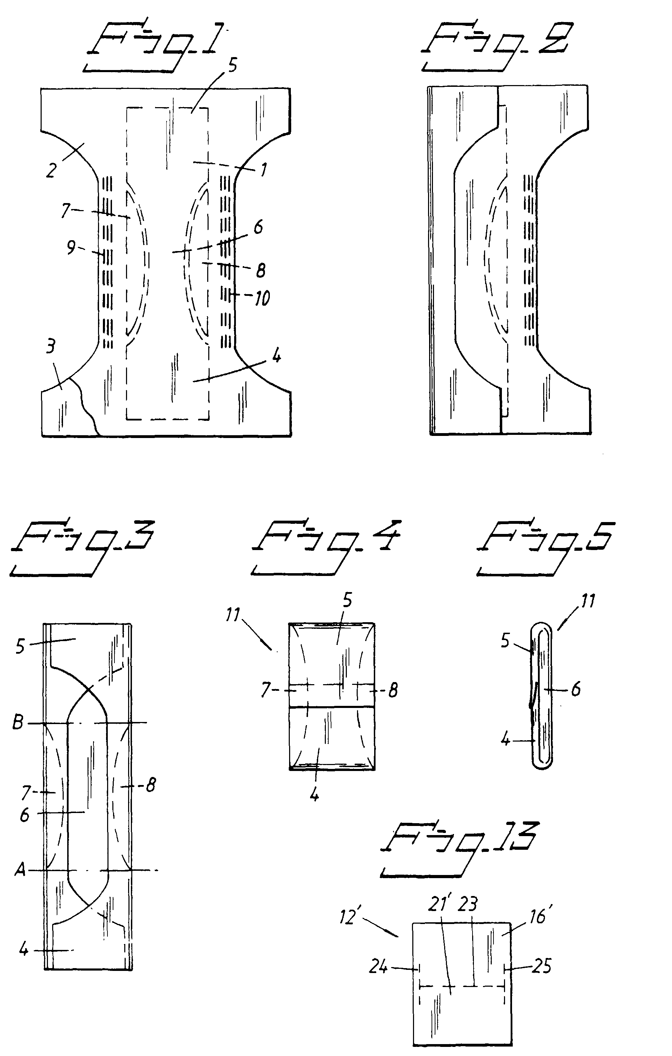

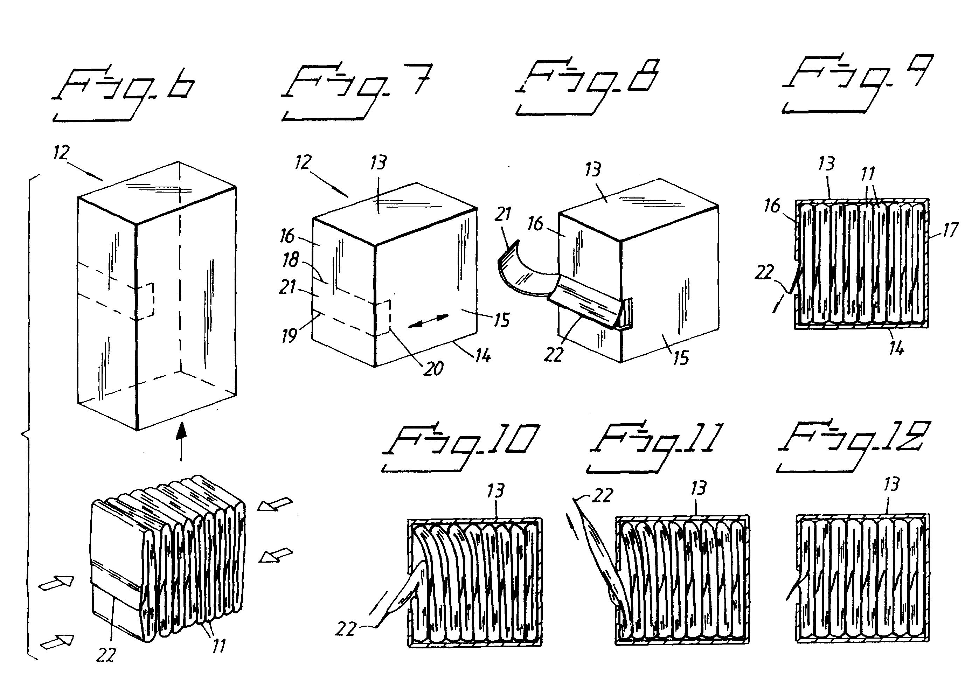

Fig. 1 illustrates from above a diaper with which the invention can be conveniently applied. The illustrated diaper includes an hourglass-shaped absorbent body 1 enclosed between two similarly shaped casing sheets 2, 3, which are joined together along parts thereof that lie outwardly of the absorbent body 1. The lateral extension of the front and rear parts 4 and 5 of the diaper is greater than that of the intermediate crotch part 6. The diaper also includes side bodies 7, 8, and elastic threads or bands 9, 10 along the long edges of the crotch part. The side bodies 7, 8 function to stabilize the three-dimensional shape of the diaper when in use, and it is desirable that these side bodies are deformed to the least possible extent when packaging the diaper after its manufacture.

It will be noted that by the longitudinal direction of the absorbent article is meant a direction that extends from the front part of the diaper to its rear part, irrespective of whether the article is package-folded or not, and irrespective of whether its lateral extension is greater than its longitudinal extension.

The diaper illustrated in Figure 1 is brought to its packaging state, i.e. package-folded, by folding the parts of the casing sheets that lie laterally outside the bodies 7, 8 in over the central parts of the diaper. Figure 2 shows the configuration of the diaper after having folded-in the lefthand parts of the casing sheets, while Figure 3 illustrates the rectangular shape of the diaper after having folded-in the right-hand parts of said sheets.

According to the invention, the front-part 4 of the article is first folded-in towards the crotch part 6 and thereafter the rear part 5, along respective transverse fold lines A and B located immediately outside the outer longitudinally extending defining points of the side bodies 7, 8. The diaper then has the configuration shown from above and in side view respectively in Figures 4 and 5. Neither the parts of the absorbent body 1 lying in the crotch part nor the side bodies 7, 8 will be affected by package-folding the diaper. A package-folded diaper of the type shown in Figure 1 is referenced 11 in Figures 4 and 5.

Naturally, package-folding of the diaper in accordance with the invention can be effected by folding-in the rear part before folding-in the front part of the diaper.

It will also be understood that the aforedescribed folding procedure can be applied with other types of absorbent articles with which it is desirable for the crotch parts of the absorbent bodies to remain in their original unfolded state or to include central non-folded parts, for one reason or another.

In the configuration illustrated in Figures 4 and 5, the inwardly folded front and rear edges of the absorbent body 1 are mutually contiguous, i.e. touch one another. However, the edge portions of the absorbent body may be folded inwardly so as to be spaced from one another instead. For instance, this latter alternative may be appropriate when parts of the diaper which are desired to be left non-folded after package-folding the diaper would be affected by package-folding with the inwardly folded edges of the absorbent body lying edge to edge.

It is also convenient to package-fold in accordance with the invention articles whose absorbent bodies have curved outer contours at the front and/or the rear parts of said bodies, therewith to achieve packaging of the articles in a generally parallelepipedic shape.

Figures 6 and 7 illustrate schematically packaging of a bundle of diapers 11 that have been package-folded to the configuration shown in Figures 4 and 5. In the Figure 6 illustration, the diaper bundle has been compressed, e.g. by holding the bundle between the arms of a gripping tool and then moving the arms towards one another until the bundle has been compressed to the extent desired. The volume of a compressed diaper bundle may be half of the volume of the bundle prior to its compression. Compression of the bundle of diapers 11 is symbolized by hollow arrows in Figure 6.

The compressed bundle of diapers 11 is inserted in some suitable manner, e.g. with the aid of the aforesaid gripping tool, into a packaging unit 12 through the open bottom thereof, as indicated by the full arrow in Figure 6. The open bottom of the packaging unit 12 is then closed and sealed, for instance by ultrasonic welding or heat welding when the packaging material is weldable, or by gluing, so as to obtain a parallelepipedic package. One such closed packaging unit 12 is shown in Figure 7. The packaging unit 12 has an upper side 13, an underside or bottom 14, two mutually opposing side walls 15, of which only one is shown in Figures 6-8, and two mutually opposing end walls 16, 17. The bundle of diapers 11 introduced into the packaging unit presses against the end walls 16, 17 and the side walls 15 are thereby subjected to a force acting in the direction of the double arrow in Figure 7. Extending across the end wall 16 are two parallel lines of perforations 18, 19, which also extend slightly into the side walls 15. A further perforated line 20 extends along the side wall 15 that faces towards the viewer of Figure 7, between the ends of the perforated lines 18, 19. The three perforated lines 18-20 together define a tear-off strip 21.

Figures 8 and 9 show the packaging unit 12 with the strip 21 torn from the end wall 16, so as to open the package. The strip 21 is suitably left connected to the side wall that cannot be seen in the Figure. As illustrated in Figures 8 and 9, the opening created by tearing of the strip 21 is located opposite the end edge 22 of the inwardly folded rear part of the first diaper 11 present in the bundle. As a result of the compressive force acting on the diaper, said end edge will be pressed out through the opening and capable of being gripped by a user.

The removal of a diaper from an opened package 12 is illustrated schematically in Figures 9-12. A diaper 11 is removed from the package, by gripping the end edge 22 accessible through said opening and then drawing said end edge downwards as indicated by arrows in Figures 9 and 10. When the upper part of the diaper has been drawn from the package, the direction in which the diaper is pulled from the package is conveniently changed, as indicated by an arrow in Figure 11. It has been found that it is easier to remove a first diaper from an inventive packaging unit than from a known packaging unit in which the diapers are exposed in a fan-like configuration.

Figure 12 illustrates the package 12 after having removed the first diaper 11 from the diaper bundle. As will be seen from this Figure, the package has an appearance which is identical to its appearance before removing the first diaper, due to the slight expansion of the remaining diapers which therewith fill up the empty space left by the first diaper removed. The pressure on the end walls 16, 17 will, however, have decreased slightly.

Although the bundle of package-folded articles are inserted through the open bottom of the packaging unit and the bottom then closed and sealed in the case of the illustrated embodiment, it will be understood that the packaging unit may be given an open top and the bundle of diapers introduced through the top of the packaging unit instead. In this latter case, an article is removed by first pulling said end edge upwards and then downwards principly in the same way as that shown in Figures 9-12 when these Figures are turned upside down.

The width of the strip 21, i.e. the vertical extension of the strip as seen in the Figures, can vary within relatively wide limits. However, the strip 21 will preferably have a smallest width of 10 mm, so as to enable the end edge 22 to be readily gripped. The upper limit of the width of the strip is determined by the need to ensure that the diapers will be retained safely in the opened package, meaning that the end wall 16 must extend unbroken from the upper side and lower side of the package 12 to some extent. The maximum width of the strip 21 shall not therefore exceed 33% of the vertical extension of the end wall 16.

Figure 13 illustrates a second embodiment of an inventive packaging unit. The sole difference between the packaging unit shown in Figure 13 and the packaging unit shown in Figures 6-12 is that the opening-forming means 21' is different to the strip 21 of the embodiment shown in Figures 6-8. The whole of the opening-forming means 21' is disposed in the end wall 16' of the packaging unit 12' and is defined by a horizontal perforated line 23 and two vertical perforated lines 24, 25 which extend parallel with the side walls of the packaging unit 12', close to the side edges of said end wall. When opening the means 21', there are formed two outwardly folded flaps between which the end of a package-folded article will protrude.

The embodiment illustrated in Figure 13 can be modified in several ways. For instance, the horizontal perforated line 23 may extend between opposing top and bottom ends of the vertical lines 24, 25. Furthermore, the vertical lines may be provided in the side walls, similar to the lines 20 in Figures 6-8. It will be understood in this regard, that the lines 20 may be provided in the end wall 16 instead of in the side walls, analogously with the lines 24, 25.

It is preferred that the opening obtained in the packaging unit has a width greater than 10 mm, suitably greater than 20 mm, conveniently greater than 30 mm and most preferably greater than 40 mm. Trials have shown that a width of 50 mm is extremely effective, and it is accordingly preferred that the opening will have a width of about 50 mm or a width that corresponds to 33% of the length of a package-folded article, depending on which of these measurements is the smallest.

It will be understood that the described and illustrated embodiments of the invention can be varied within the scope of the inventive concept, particularly with regard to the shape or configuration of the absorbent articles. Furthermore, other types of weakening lines than perforated lines can be used to define the tear-off strip. The packaging unit may also contain more than one bundle of articles placed horizontally adjacent one another and/or stacked on top of one another. When the bundles are placed horizontally adjacent one another, the tear-off strip may conveniently be common to mutually adjacent bundles, therewith facilitating manufacture of such a packaging unit. Furthermore, the openable part of the end wall may be provided at the opening-forming end with a grip tab or the like to facilitate opening of the packaging unit. The invention is therefore restricted solely by the contents of the following Claims.

## Claims

1. A flexible packaging unit (12) containing at least one compressed bundle of absorbent articles (11), such as diapers or incontinence guards, each comprising a rear part, a front part, an intermediate crotch part, and an absorbent body enclosed between casing sheets, wherein said articles are folded so that at least longitudinally outer portions of the front and the rear parts will be folded inwardly towards the crotch part and face in one and the same direction in said bundle, wherein the packaging unit encloses the bundle or bundles of articles (11) and has an essentially parallelepipedic shape with an upper side (13), an underside (14), two mutually opposing side walls (15) and two mutually opposing end walls (16, 17), and opening-forming means (21) for enabling articles (11) to be removed from the packaging unit, **characterized** in that the opening-forming means is comprised of an opening-forming part (21) of the end wall (16) towards which the inwardly folded portions of the front and rear parts of the articles (11) in said bundle face, wherein the opening-forming part (21) extends parallel with the front and rear edges of the inwardly folded front and rear portions of the articles, wherein said opening-forming part (21) can be moved out of the plane of said end wall (16), and wherein said opening-forming part is so positioned and has such a width that at least one (22) of the outer edges of the front or the rear part of the article will be exposed when moving the opening-forming part (21) out of the plane of said end wall.

2. A packaging unit according to Claim 1, in which at least one of the front or rear parts of the absorbent articles (11) includes a longitudinally outer edge part of casing material outwardly of the absorbent body which overlaps a part of the upper front or rear part of said article in the package-folded state thereof, **characterized** in that the opening-forming part (21) extends along the edge (22) of the outer edge part of said casing material.

3. A packaging unit according to Claim 1 or Claim 2, **characterized** in that the opening-forming part (21) has a smallest width of 10 mm and a largest width which corresponds to 33% of the length of a package-folded article (11).

4. A packaging unit according to any one of Claims 1-3, **characterized** in that the opening-forming part (21) is defined by two mutually parallel weakening lines (18, 19) which extend across the full width of said end wall, from one side wall (15) to the other.

5. A packaging unit according to Claim 4, **characterized** in that the parallel weakening lines (18, 19) extend slightly into the side walls (15); and in that a further weakening line (20) is provided in one of said side walls, between the ends of the mutually parallel weakening lines.

6. A packaging unit according to any one of Claims 1-3, **characterized** in that the opening-forming part (21') is defined by a first weakening line (23) which extends over at least the major part of the width of the end wall (16'), and two second, mutually parallel weakening lines (24, 25) which extend perpendicularly to the first weakening line on respective sides thereof.

7. A packaging unit according to any one of Claims 1-6, **characterized** in that the whole of the opening-forming part (21') is located in the end wall (16') towards which the inwardly folded portions of the front and rear parts of the articles in said bundle face.

## Patentansprüche

1. Flexible Verpackungseinheit mit wenigstens einem komprimierten Bündel von Absorptionsartikeln (11), wie z.B. Windeln oder Inkontinenzschutzeinrichtungen, die jeweils einen hinteren Teil, einen vorderen Teil, einen Zwischen-Schrittteil und einen Absorptionskörper aufweisen, der zwischen Mantellagen eingeschlossen ist, wobei die Artikel derart gefaltet sind, dass wenigstens in Längsrichtung äußere Abschnitte des vorderen und hinteren Teiles nach innen in Richtung des Schrittteiles gefaltet sind und in ein und dieselbe Richtung in dem Bündel gerichtet sind, wobei die Verpackungseinheit das oder die Bündel von Artikeln (11) umschließt und eine im Wesentlichen quaderförmige Form mit einer Oberseite (13), einer Unterseite (14), zwei gegenüberliegenden Seitenwänden (15) und zwei gegenüberliegenden Endwänden (16, 17) und eine Öffnungsausbildungseinrichtung (21) zum Ermöglichen, dass Artikel (11) von der Verpackungseinheit entnommen werden, aufweist,
dadurch **gekennzeichnet,** dass
die Öffnungsausbildungseinrichtung aus einem Öffnungsausbildungsteil (21) der Endwand (16) besteht, zu der die nach innen gefalteten Abschnitte des vorderen und hinteren Teiles der Artikel (11) in dem Bündel gerichtet sind, wobei der Öffnungsausbildungsteil (21) sich parallel mit dem vorderen und hinteren Rand der nach innen gefalteten vorderen und hinteren Abschnitte der Artikel erstreckt, wobei der Öffnungsausbildungsteil (21) aus der Ebene der Endwand (16) bewegt werden kann, und wobei der Öffnungsausbildungsteil derart angeordnet ist und eine derartige Breite aufweist, dass wenigstens einer (22) der äußeren Ränder des vorderen und hinteren Teiles des Artikels freigelegt ist, wenn der Öffnungsausbildungsteil (21) aus der Ebene der Endwand bewegt wird.

2. Verpackungseinheit nach Anspruch 1, bei welcher der vordere und/oder hintere Teil der Absorptionsartikel (11) einen in Längsrichtung äußeren Randteil aus Mantelmaterial außerhalb des Absorptionskörpers aufweist, der einen Teil des oberen vorderen oder hinteren Teils des Artikels in dem zum Verpacken gefalteten Zustand desselben überlappt,
dadurch **gekennzeichnet,** dass
der Öffnungsausbildungsteil (21) sich entlang des Randes (22) des äußeren Randteiles des Mantelmaterials erstreckt.

3. Verpackungseinheit nach Anspruch 1 oder 2,
dadurch **gekennzeichnet,** dass
der Öffnungsausbildungsteil (21) eine geringste Breite von 10 mm und eine größte Breite, die 33% der Länge des zum Verpacken gefalteten Artikels (11) entspricht, aufweist.

4. Verpackungseinheit nach einem der Ansprüche 1 bis 3,
dadurch **gekennzeichnet,** dass
der Öffnungsausbildungsteil (21) durch zwei zueinander parallele Schwächungslinien (18, 19) definiert ist, die sich über die volle Breite der Endwand von der einen Seitenwand (15) zu der anderen erstrecken.

5. Verpackungseinheit nach Anspruch 4,
dadurch **gekennzeichnet,** dass
die parallelen Schwächungslinien (18, 19) sich ein wenig in die Seitenwände (15) erstrecken; und dadurch, dass eine weitere Schwächungslinie (20) in einer der Seitenwände vorgesehen ist, und zwar zwischen den Enden der zueinander parallelen Schwächungslinien.

6. Verpackungseinheit nach einem der Ansprüche 1 bis 3,
dadurch **gekennzeichnet**, dass
der Öffnungsausbildungsteil (21') durch eine erste Schwächungslinie (23), die sich über wenigstens den wesentlichen Teil der Breite der Endwand (16') erstreckt, und durch zwei zweite zueinander parallele Schwächungslinien (24, 25) definiert ist, die sich senkrecht zu der ersten Schwächungslinie an jeweiligen Seiten derselben erstrecken.

7. Verpackungseinheit nach einem der Ansprüche 1 bis 6,
dadurch **gekennzeichnet,** dass
der gesamte Öffnungsausbildungsteil (21') in der Endwand (16') ausgebildet ist, zu der die nach innen gefalteten Abschnitte des vorderen und hinteren Teiles der Artikel in dem Bündel gerichtet sind.

## Revendications

1. Elément d'emballage souple (12) contenant au moins un ballot comprimé d'articles absorbants (11), tels que des couches-culottes ou des protections contre l'incontinence, chacun de ces articles comprenant une partie arrière, une partie avant, une partie intermédiaire d'entrejambes, et un corps absorbant enfermé entre des feuilles d'enveloppe, dans lequel lesdits articles sont pliés de telle manière qu'au moins des portions longitudinalement extérieures des parties avant et arrière sont pliées vers l'intérieur vers la partie d'entrejambes et sont tournés dans une seule et même direction dans ledit ballot, dans lequel l'élément d'emballage renferme le ou les ballots d'articles (11) et a une forme globalement parallélépipédique comportant une face supérieure (13), une face inférieure (14), deux parois latérales (15) mutuellement opposées et deux parois d'extrémité (16, 17) mutuellement opposées, et un moyen (21) pour former une ouverture pour permettre aux articles (11) d'être retirés de l'emballage, **caractérisé** en ce que le moyen pour former une ouverture est constitué d'une partie (21) de la paroi d'extrémité (16) pour former une ouverture vers laquelle sont tournées les portions pliées vers l'intérieur des parties avant et arrière des articles (11) dudit ballot, dans lequel la partie (21) pour former une ouverture s'étend parallèlement aux bords avant et arrière des portions pliées vers l'intérieur avant et arrière des articles, dans lequel ladite partie (21) pour former une ouverture peut être retirée du plan de ladite paroi d'extrémité (16), et dans lequel ladite partie pour former une ouverture a une position et une largeur telles qu'au moins un (22) des bords extérieurs de la partie avant ou arrière de l'article se retrouve exposé lorsque l'on retire du plan de ladite paroi d'extrémité la partie (21) pour former une ouverture.

2. Elément d'emballage selon la revendication 1, dans lequel au moins l'une des parties avant ou arrière des articles absorbants (11) comprend une partie de bord longitudinalement extérieure de matériau d'enveloppe à l'extérieur du corps absorbant qui chevauche une partie de la partie supérieure avant ou arrière dudit article dans l'état plié en emballage de ce dernier, **caractérisé** en ce que la partie (21) pour former une ouverture s'étend le long du bord (22) de la partie de bord extérieure dudit matériau d'enveloppe.

3. Elément d'emballage selon la revendication 1 ou 2, **caractérisé** en ce que la partie (21) pour former une ouverture a une largeur minimale de 10 mm et une largeur maximale qui correspond à 33 % de la longueur d'un article (11) plié en emballage.

4. Elément d'emballage selon l'une quelconque des revendications 1 à 3, **caractérisé** en ce que la partie (21) pour former une ouverture est définie par deux lignes favorisant la rupture (18, 19), parallèles entre elles, qui s'étendent sur toute la largeur de ladite paroi d'extrémité, d'une paroi latérale (15) à l'autre.

5. Elément d'emballage selon la revendication 4, **caractérisé** en ce que les lignes parallèles favorisant la rupture (18, 19) s'étendent légèrement dans les parois latérales (15); et en ce qu'une ligne supplémentaire favorisant la rupture (20) est prévue dans l'une desdites parois latérales, entre les extrémités des lignes favorisant la rupture parallèles entre elles.

6. Elément d'emballage selon l'une quelconque des revendications 1 à 3, **caractérisé** en ce que la partie (21') pour former une ouverture est définie par une première ligne favorisant la rupture (23) qui s'étend sur au moins la majeure partie de la largeur de la paroi d'extrémité (16'), et deux deuxièmes lignes favorisant la rupture (24, 25) parallèles entre elles qui s'étendent perpendiculairement à la première ligne favorisant la rupture sur des côtés respectifs de celle-ci.

7. Elément d'emballage selon l'une quelconque des revendications 1 à 6, **caractérisé** en ce que la totalité de la partie (21') pour former une ouverture est située dans la paroi d'extrémité (16') vers laquelle sont tournées les portions pliées vers l'intérieur des parties avant et arrière des articles dudit ballot.
